# EUROPEAN PATENT APPLICATION

(11) **EP 4 825 257 A1**
(43) Date of publication of application: **21.10.2026**
(21) Application number: 25305546.1
(22) Date of filing: 15.04.2025
(51) Int. Cl.: A45D 34/00, A45D 40/00, A61K 8/27, A61Q 3/02, B65D 23/08, B65D 81/30

(54) **COSMETIC CONTAINER WITH PROTECTION AGAINST UV/HEV-LIGHT**

(71) Applicant: Nexdot, 93230 Romainville (FR)
(72) Inventor: D'AMICO, Michele, 93230 Romainville (FR); WROBLEWSKI, Claire, 93230 Romainville (FR); CARRU, Milène, 93230 Romainville (FR); DUBERTRET, Benoit, 93230 Romainville (FR)
(74) Representative: Icosa

(57) **Abstract**

The present invention relates to a cosmetic bottle carrying a filtering film allowing protection of radiation curable composition sensitive to UV/HEV-light.

## Description

### FIELD OF DISCLOSURE

The present disclosure relates to a cosmetic bottle with a filtering film absorbing ultraviolet (UV) or high-energy visible (HEV) light and containing a radiation curable composition. This curable composition comprises a photoinitiator. The filtering film prevents the curable composition from curing or gelling during storage - even in natural light storage - while providing visibility of the colour of the radiation curable composition. The disclosure is especially directed to UV nail polish or gel.

### BACKGROUND OF DISCLOSURE

Typical radiation curable cosmetic composition like gel nail polish formulations include methacrylate monomers and a photoinitiator. Upon exposure to UV/HEV light, the radiation curable composition cures, *i.e.,* undergoes polymerization and solidification. Curing wavelengths typically range between 340 nm to 380 nm for UV light, but HEV light, especially in a range between 380 nm and 420 nm, also induces photopolymerization. However, upon exposure to light over time, the radiation curable cosmetic composition cures, gels and develops clumps in its storage vessel or bottle.

Accordingly, radiation curable composition cannot be stored in clear or transparent bottles, and thus an external color indicator or description must be used in order to notify the consumer of the color of the product. Therefore, conventional bottles must be tinted or opaque to block UV/HEV light, in order to mitigate or prevent curing of the composition during storage. There is thus a need in the art for a system providing a clear or transparent bottle containing radiation curable composition so that the true color, tint and pigment complexion of the composition is readily visible to a user. At the same time, this clear or transparent bottle should block unwanted UV/HEV light.

US patent application US20220192343 addresses this problem and suggests using a photoinitiator having an absorption wavelength in the UV-B range or UV-A2 range, namely below 330 nm, and a clear UV blocking coating on the bottle. However, this solution has various drawbacks. The UV light required to cure the curable composition is almost not present in natural light: the curable composition cannot cure upon exposure to sunshine - at least in a time acceptable for the user. Thus, artificial lighting is required, which limits the use of such compositions to cosmetic salons like nail salons. In addition, this solution does not allow the use of very efficient photoinitators such as phosphine oxides, well known for the absence of residual color after cure and their ability to provide depth curing for thick coatings. Indeed, a composition comprising 2,4,6-trimethylbenzoylethoxyphenylphosphine oxide as photoinitiator resulted in a cloudy product after three days of storage in a glass container.

The object of this disclosure is to overcome these drawbacks and improve the trade off in the choice of photoinitiators in radiation curable compositions and the ability to have a transparent cosmetic bottle allowing the consumer to perceive the true color of the composition.

### SUMMARY

This disclosure thus relates to a cosmetic bottle comprising a glass container, filled with a radiation curable composition and covered by a filtering film. The radiation curable composition comprises at least 500 ppm of a photoinitiator having a cleaving maximum λ_{clm} in a range from 350 nm to 400 nm, and an absorption function A_{φ}(λ). The filtering film comprises semi-conductive nanoparticles absorbing UV-light, said filtering film having an absorption function A_{ff}(λ) and a wavelength of transition λ_{cut} in a range from 370 nm to 420 nm. Finally, the filtering film lowers by 95% or more the power absorbed by the photoinitiator when exposed to a light source having an irradiance P(λ), as defined by ratio α in equation (I): $α = \frac{{\int }_{300}^{500} P \left(λ\right) ⋅ {10}^{- {A}_{ff} \left(λ\right)} ⋅ \left(1-{10}^{- {A}_{φ} \left(λ\right)}\right) dλ}{{\int }_{300}^{500} P \left(λ\right) ⋅ \left(1-{10}^{- {A}_{φ} \left(λ\right)}\right) dλ} \leq 0.05$ (I)

In an embodiment, the photoinitiator is selected from the group consisting of phosphinate or phosphine oxide, preferably from the group consisting of 2,4,6-trimethylbenzoylethoxyphenylphosphine oxide, diphenyl(2,4,6-trimethylbenzoyl)phosphine oxide, phenylbis(2,4,6-trimethylbenzoyl)phosphine oxide, bis(2,6-dimethoxybenzoyl)(2,4,4-trimethylpentyl)phosphine oxide, and mixtures thereof.

In an embodiment, the semi-conductive nanoparticles have a formula

MₓE_{y} (IV),

wherein:
M is selected from the group consisting of Zn, Cd, Hg, Cu, Ag, Al, Ga, In, Si, Ge, Sn, Pb or a mixture thereof;
E is selected from the group consisting of S, Se, Te, N, P, As, Sb, or a mixture thereof; x and y are independently a decimal number from 0 to 5; and
x and y are not simultaneously equal to 0.

In particular, the semi-conductive nanoparticles comprise a material selected from the group consisting of CdS, CdSe, CdTe, ZnS, ZnSe, ZnTe, HgS, HgSe, HgTe, GeS, GeSe, GeTe, SnS, SnSe, SnTe, PbS, PbSe, PbTe, GeS₂, GeSe₂, SnS₂, SnSe₂, CuInS₂, CuInSe₂, CuAlS₂, CuAlSe₂, CuInZnS, CuInZnSe, AgInS₂, AgInSe₂, CuS, Cu₂S, Ag₂S, Ag₂Se, Ag₂Te, FeS, FeS₂, InP, Cd₃P₂, Zn₃P₂, AIN, AlP, AlAs, AlSb, GaN, GaP, GaAs, GaSb, InN, InP, InAs, InSb, InAsP, or a mixture thereof; preferably selected from the group consisting of CdS, CdSe, CdTe, ZnS, ZnSe, ZnTe, or a mixture thereof.

More particularly, the semi-conductive nanoparticles are core/shell nanoparticles selected from the group consisting of ZnSeₓS₍₁₋ₓ₎/ZnSe_{y}S_{(1-y)}, where x and y are rational numbers between 0 (included) and 1 (included).

In an embodiment, the ratio α is less than 0.02, preferably less than 0.01, more preferably less than 0.005.

In an embodiment, the absorbance A_{ff}(k) of the filtering film has:
- a local maximum absorbance of highest wavelength in the range from 370 nm to 420 nm, said local maximum having an absorbance value Aₘₐₓ for a wavelength λₘₐₓ,
- a value of 0.9Aₘₐₓ for a wavelength λ_{0.9}, k_{0.9} being greater than λₘₐₓ;
- a value of 0.5Aₘₐₓ for a wavelength λ_{0.5}, λ_{0.5} being greater than λ_{0.9}; and
- |λ_{0.5} - k_{0.9}| is less than 15 nm.

In an embodiment, the weight concentration of the semi-conductive nanoparticles in the filtering film is in a range from 5 wt% to 50 wt%, based on the weight of the filtering film, for a 20 µm-thick film.

In an embodiment, the semi-conductive nanoparticles are encapsulated in an encapsulating material, thereby forming composite particles.

In an embodiment, the filtering film is transparent, preferably transparent and uncoloured.

In an embodiment, the glass container has a colour (L*u, b*u) and the difference of lightness between the glass container and the glass container with the filtering film is lower than 5. In particular, the difference Δb*=|b*c-b*u| of the parameter b* of the glass container - b*u - and the glass container with the filtering film - b*c - is lower than 20, preferably lower than 15.

In an embodiment, the radiation curable composition is a nail varnish, a nail polish or a gel nail coating.

In an embodiment, the glass container carries the filtering film over a predetermined surface and carries an opaque treatment outside said predetermined surface. The predetermined surface behaves like a window allowing the user to visualize the true colour of the radiation curable composition.

### DEFINITIONS

In the present disclosure, the following terms have the following meanings:

"**Absorbance**" is the decimal logarithm of ratio I₀/I, where I₀ is the intensity of light incident on a sample and I is the intensity of light transmitted through said sample. In this disclosure, absorbance is measured for wavelengths in UV and visible range from 300 nm to 780 nm. For solid samples (coating), absorbance is measured for a sample of specified thickness. For liquid samples (solutions of absorbing compound), absorbance is measured in a 1-centimeter light path cuvette. Absorbance of 1 means that 9 out of 10 photons are absorbed by the sample. Absorbance of 0.3 means that 1 out of 2 photons is absorbed by the sample.

"**Color space**": refers to a model for representation of color perceived by observers. In this disclosure, Color space refers to the CIE L*a*b* color space - also known as L*a*b* - defined by the International Commission on Illumination (CIE) in 1976. In CIE L*a*b*, a colour is represented by lightness (L*), position between red and green (a*) and position between yellow and blue (b*). Within this model, all colours for a given lightness can be represented within a circle, in which a* and b* are coordinates of colours.

"**Colorimetric coefficients**": refer to chroma and hue of a colour, in the international colorimetric system CIE L*a*b* (1976), and are calculated between 380 and 780 nm, taking the standard illuminant D65 and the observer into account (angle of 2°). The observer is a "standard observer" as defined in the international colorimetric system CIE L*a*b*.

"**HEV light**" refers to high-energy visible light which is short-wave light in the violet/blue band from 380 nm to 450 nm in the visible spectrum.

"**Lightness**"; refers to the absolute brightness value of light. In CIE LAB colorimetric space, Lightness ranges from L* = 0 (black) to L* = 100 (diffuse white).

"**Luminous transmission"**: refers to an average in the 380-780 nm wavelength range that is weighted according to the sensitivity of the eye at each wavelength of the range and measured under D65 illumination conditions (daylight), as defined in the standard ISO 13666:1998.

"**Nanometric** si**z**e" refers to a size of matter in which quantum effects appear due to confinement. For semi-conductive nanoparticles, nanometric size has to be defined with the average Bohr radius of an electron/hole pair. Confinement is effective for size in at least one dimension of nanoplates below 10 nm, preferably below 5 nm. Confinement is effective for section of nanorods below 100 nm², preferably below 50 nm². Confinement is effective for diameter of nanospheres below 20 nm, preferably below 15 nm, more preferably below 10 nm.

"**Nanoparticle**" refers to a particle having a size in at least one of its dimensions below 100 nm. For a nanosphere, diameter should be below 100 nm. For a nanoplate, thickness should be below 100 nm. For a nanorod, diameter should be below 100 nm.

"**Semi-conductive nanoparticles**" refers to particles made of a material having an electronic structure corresponding to semi-conductive materials known in electronic industry but having a **nanometric size**. Due to their specific electronic structure, semi-conductive materials behave as high-pass absorbing materials. Indeed, light having a wavelength more energetic than band gap may be absorbed by the semi-conductive material, yielding an electron/hole pair, an exciton, which later recombine in the material and dissipate heat, or emit light, or both. On the contrary, light having a wavelength less energetic than band gap cannot be absorbed: semi-conductive material is transparent for these wavelengths. In macroscopic semi-conductive materials, visible light is generally absorbed while near/mid infra-red light is not absorbed. When semi-conductive particles have a **nanometric size**, confinement - *i.e.,* shape and nanometric size - governs electronic structure following the rules of quantum mechanics and light absorption may be limited to UV range or UV and high energy visible light.

"**Transparent**" refers to a material with two properties. First, light scattering by the material should be low, typically below 1% as measured with standard haze measurement according to ASTM D1003- 00, preferably below 0.8%, even preferably below 0.5%. Second, the shape of an object seen throughout the material should be unaltered, in the sense that a consumer can recognize an object when looking through the material. In this disclosure, transparency is not related to the absorbance of visible light: a material may be transparent and coloured. Optionally, the material is uncoloured when the absorbance of the material is less than 0.05 for a range of wavelength from 420 nm to 780 nm, preferably from 400 nm to 780 nm, more preferably for the whole visible range: from 380 nm to 780 nm. With such low absorbance, there is no attenuation effect visible by eye, nor change in colour perception: the material is transparent and uncoloured. The material may be a glass container, a plastic container or a film.

"**UV-Light**": refers to electromagnetic radiations having a wavelength comprised between 280 nm and 380 nm. In this disclosure UV-C light having wavelength below 280 nm is not considered.

"**Visible light**" refers to electromagnetic radiations having a wavelength comprised between 380 nm and 780 nm.

"**wt**%" refers to the weight percentage of a component in a blend or a formulation, based on the weight of the solid blend - after drying or cure, as the case may be.

### DETAILED DESCRIPTION

This disclosure relates to a cosmetic bottle filled with a radiation curable composition.

### Cosmetic bottle

The cosmetic bottle comprises a glass container. The composition of glass and the shape of the glass container may vary according to the usual practices in cosmetic bottles manufacturing. In an embodiment, the glass container is transparent and uncoloured - often referred to as white glass in the industry. Alternatively, the glass container may be coloured - evenly or with patterns or images. Notwithstanding its colour, a glass container may require protection against UV/HEV-light.

The cosmetic bottle contains a curable composition.

### Radiation curable composition

The radiation curable composition comprises at least one monomer or oligomer; and at least one photoinitiator for initiating the polymerization of said monomer or oligomer.

The monomer or oligomer are conventional compounds, mainly based on (meth)acrylic compounds. In this disclosure, a (meth)acrylic monomer or (meth)acrylic oligomer is a compound comprising having acrylic or methacrylic groups. (Meth)acrylates may be monofunctional (meth)acrylates or multifunctional (meth)acrylates. Functional (meth)acrylates include urethane (meth)acrylates; reaction products of (meth)acrylic acid with polyols, especially derivatives of glycol, glycerol or pentaerythritol. Acryloyl derivatives such as acryloyl morpholine are also included in the (meth)acrylic compounds. Resins obtained from (meth)acrylic compounds are also included in the (meth)acrylic compounds.

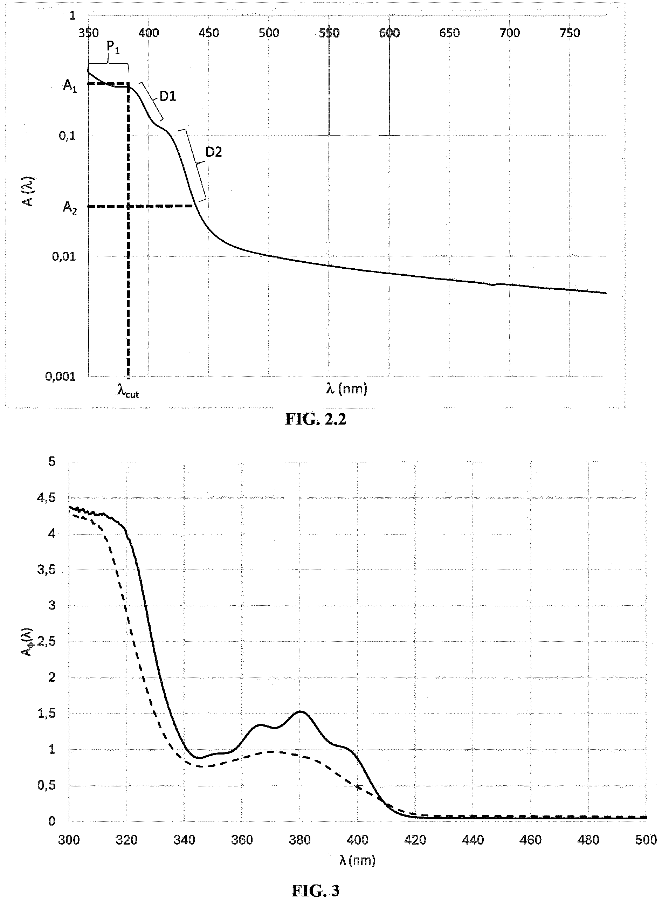

The photoinitiator is a free radical initiator, *i.e.,* a compound that generates one - or more often two - free radicals after absorption of an exciting photon of energy associated with the electronic orbital structures of the photoinitiator. A band of wavelength is thus active to promote the photinitiator in an excited state, leading to cleavage of the photoinitiator. Therefore, it is necessary to define the range of wavelengths which is active, *i.e.,* absorbed by the photoinitiator and may lead to cure initiation. The absorption curve A_{φ}(λ) of the photoinitiator - also referred to as absorption function, and illustrated on figure 3 with diphenyl(2,4,6-trimethylbenzoyl)phosphine oxide in solid line - defines this active range of wavelengths - roughly from 350 nm to 410 nm on figure 3 - with a maximum of absorption. In this disclosure, photoinitiators are excited by photon in a band of wavelength centred around a wavelength of maximum cleavage λ_{clm}, with λ_{clm} comprised in a range from 350 nm to 400 nm. The width of the absorption band is typically comprised between 50 nm and 100 nm.

In the disclosure, the radiation curable composition contains at least 1000 ppm of photoinitiator. Higher concentration is also suitable. Weight concentration greater than 0.5wt%, or even 1wt% may be used.

These specific photoinitiators are thus activated by natural sunlight, which is desired for curable composition intended to cure by simple exposure to light, which is often the case for cosmetics like nail varnishes, nail polish or gel nail coatings for instance. However, these photoinitiators may be also activated during storage, depending on light conditions, and lead to a degradation of the curable composition: typically, a nail varnish will become too viscous for application, or even totally solid like.

Particularly suitable photoinitiators are selected from the group of phosphinates or phosphine oxides. The phosphine oxides have a small absorption band in the long wave UV/HEV around 350 nm to 420 nm, which makes them ideally suited to cure at depth. Their non-yellowing, photobleaching properties contribute to good through cure by allowing the UV/HEV light to penetrate as cure proceeds. Although the phosphine oxides are yellow materials, any remaining phosphine oxide in the cured coating will photobleach on further exposure to UV/HEV or daylight to give excellent whites. The phosphine oxides are especially suitable for highly pigmented formulations laid in thick films.

Among phosphine oxides, (2,4,6-trimethylbenzoyl)ethoxyphenylphosphine oxide, diphenyl(2,4,6-trimethylbenzoyl)phosphine oxide, phenylbis(2,4,6-trimethylbenzoyl)phosphine oxide, and bis(2,6-dimethoxybenzoyl)(2,4,4-trimethylpentyl)phosphine oxide are especially suitable.

The radiation curable composition may further comprise additives in conventional proportions. These additives include stabilizers such as antioxidants, UV light absorbers, light stabilizers, anti-yellowing agents, adhesion promoters, dyes, pigments, rheology modifiers, lubricants, cross-linking agents. They should neither decrease the effectiveness of polymerization nor deteriorate optical properties of the cured composition.

The radiation curable composition may further comprise a solvent, provided that polymerization is not hindered by the solvent. Solvent may be selected from ethylacetate, n-butylacetate or isopropanol for instance.

### Filtering film

In the disclosure, the cosmetic bottle comprises a filtering film. Preferably, the glass container is coated with the filtering film.

The aim of the filtering film is to prevent light reaching the photoinitiator intended to initiate cure of the radiation curable composition. To this end, the filtering film comprises semi-conductive nanoparticles absorbing UV/HEV light.

### Absorption curve of the filtering film

Semi-conductive nanoparticles bring especially interesting light absorbing properties to filtering films comprising them. In particular, with proper selection of composition and structure of semi-conductive nanoparticles, filtering films having a sharp transition between range of absorbed light (of high energy) and range of transmitted light (low energy) may be designed.

Due to their electronic structure, semi-conductive nanoparticles behave as high pass filters: absorbance is high for wavelength of high energy, *i.e.,* short wavelengths. On the contrary, absorbance for wavelength of low energy, *i.e.,* long wavelengths, is low. The transition between both domains of high and low absorbance may be defined by the wavelength λₘₐₓ defined as follow: λₘₐₓ corresponds to the local maximum absorbance of highest wavelength in the range from 350 nm to 420 nm. It is noteworthy that semi-conductive nanoparticles absorbing UV-A light are also intrinsically absorbing UV-B light. This is a major difference with organic UV-A absorbers which present a peak of absorption, without absorption below and above the peak.

In other words, light of wavelength less than the wavelength λₘₐₓ will not be transmitted whereas light of wavelength greater than the wavelength λₘₐₓ will be transmitted. In other words, light initiating cure of the radiation curable composition will not be transmitted whereas visible light will be transmitted, in order to prevent any undesired coloration of the filtering film.

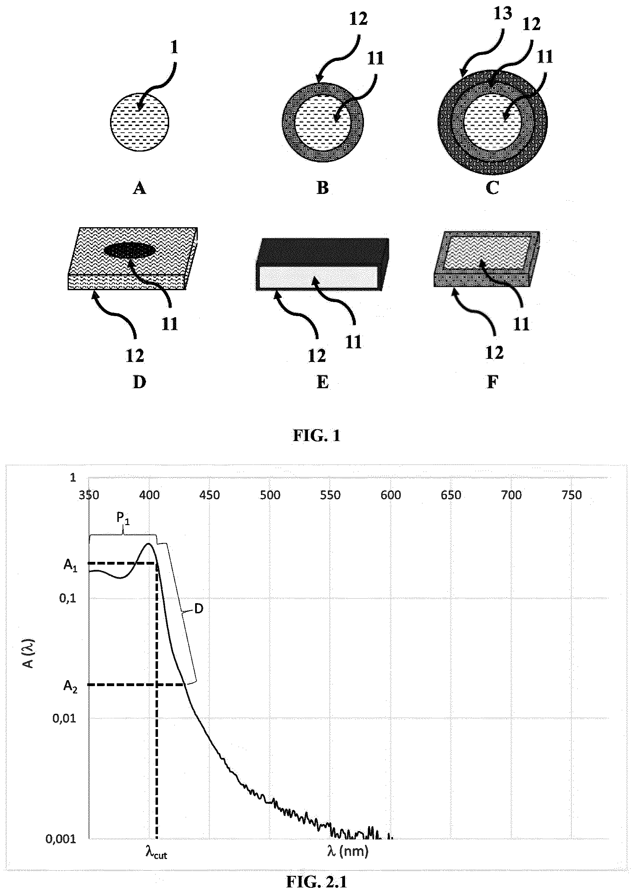

Figure 2.1 shows the generic absorbance curve of a light filtering coating as a function of wavelength of light from 350 nm to 780 nm: A_{ff}(λ). The absorbance curve presents three zones. In low wavelength area, *i.e.* in UV/HEV light, absorbance is high and/or is roughly constant, defining a first plateau P₁ with an average absorbance A₁. After first plateau P₁, absorbance decreases sharply to reach a value A₂ of a tenth of A₁: *A*₂ = *A*₁/10, thus defining a decreasing zone D. The limit between plateau P₁ and decreasing zone D defines the wavelength of transition λ_{cut}. After the decreasing zone D, absorbance may decrease and/or stabilize in a second plateau P₂, extending up to red end of visible light, namely 780 nm.

In this disclosure, the filtering film has a wavelength of transition λ_{cut} in a range from 370 nm to 420 nm.

The width of decreasing zone D is generally less than 100 nm, preferably less than 50 nm, more preferably less than 40 nm, even preferably less than 30 nm.

While absorbance curves always have this generic shape, details vary with the nature of materials used and exact determination of λ_{cut} may be difficult.

In some embodiments, absorbance curve shows a clear maximum at the limit of P₁, and D, as shown on Figure 2.1. This maximum corresponds to λₘₐₓ. In this embodiment, λ_{cut} may be defined by the following formula, where λ_{cut} is in the decreasing zone D: $A \left({λ}_{cut}\right) = \frac{{\int }_{350}^{{λ}_{cut}} A \left(λ\right) d λ}{{λ}_{cut} - 350}$

In this embodiment, λ_{cut} may be defined alternatively by the following formula for a local maximum: ${\left.\frac{\partial log A}{\partial λ}\right)}_{{λ}_{cut}} = 0$

In other embodiments, absorbance curve is monotonously decreasing slowly before decreasing sharply, as shown on Figure 2.2. In this embodiment, λ_{cut} may be defined by the lowest value of λ where decrease of absorbance becomes significant in decreasing zone, for instance where: $\frac{\partial log A}{\partial λ} < - 0 , 01$

The different determinations of λ_{cut} proposed above give different but near values. In this disclosure, a value of λ_{cut} has to be considered as a rounded value with uncertainty of ± 5 nm.

Physically, λ_{cut} corresponds to the lowest energy of an exciton in the semi-conductive nanoparticle.

In one embodiment, absorbance A_{ff}(k) of the filtering film has:
- a local maximum absorbance of highest wavelength in the range from 370 nm to 420 nm, said local maximum having an absorbance value Aₘₐₓ for a wavelength λₘₐₓ,
- a value of 0.9Aₘₐₓ for a wavelength λ_{0.9}, λ_{0.9} being greater than λₘₐₓ;
- a value of 0.5Aₘₐₓ for a wavelength 1₀.₅, 1₀.₅ being greater than λ_{0.9}; and
- |λ_{0.5} - λ_{0.9}| is less than 15 nm.

Physically, λₘₐₓ corresponds to the peak of absorption leading to an exciton in the semi-conductive nanoparticle. Thus, λₘₐₓ is necessarily less than - but close to - λ_{cut}.

In a preferred configuration, |λ_{0.5} - k_{0.9}| is less than 10 nm, or less than 5 nm.

In an embodiment, absorbance of said light filtering material has a value of 0.1Aₘₐₓ for a wavelength λ_{0.1}, λ_{0.1} being greater than λ_{0.9}; and wherein |λ_{0.1} - λ_{0.9}| is less than 30 nm, preferably less than 20 nm, more preferably less than 15 nm.

### Semi-conductive nanoparticles

The wavelength λₘₐₓ of the semi-conductive nanoparticles can be adjusted depending on the composition, shape, dimensions and direct environment of the semi-conductive nanoparticles.

Especially suitable semi-conductive nanoparticles have a formula

MₓE_{y} (IV),

wherein:
M is selected from the group consisting of Zn, Cd, Hg, Cu, Ag, Al, Ga, In, Si, Ge, Sn, Pb or a mixture thereof;
E is selected from the group consisting of S, Se, Te, N, P, As, Sb, or a mixture thereof; x and y are independently a decimal number from 0 to 5; and
x and y are not simultaneously equal to 0.

In a specific embodiment, semi-conductive nanoparticles comprise a material selected from the group consisting of CdS, CdSe, CdTe, ZnS, ZnSe, ZnTe, HgS, HgSe, HgTe, GeS, GeSe, GeTe, SnS, SnSe, SnTe, PbS, PbSe, PbTe, GeS₂, GeSe₂, SnS₂, SnSe₂, CuInS₂, CuInSe₂, CuAlS₂, CuAlSe₂, CuInZnS, CuInZnSe, AgInS₂, AgInSe₂, CuS, Cu₂S, Ag₂S, Ag₂Se, Ag₂Te, FeS, FeS₂, InP, Cd₃P₂, Zn₃P₂, AIN, AlP, AlAs, AlSb, GaN, GaP, GaAs, GaSb, InN, InP, InAs, InSb, InAsP, or a mixture thereof

In this disclosure, semi-conductive nanoparticles may have different shapes, provided that they present a nanometric size leading to confinement of exciton created in the nanoparticle. Semi-conductive nanoparticles may be nanospheres, nanoplates or nanorods.

Semi-conductive nanoparticles may have nanometric sizes in three dimensions, allowing quantum confinement in all three spatial dimensions. Such semi-conductive nanoparticles are for instance nanocubes or nanospheres.

Semi-conductive nanoparticles may have nanometric sizes in two dimensions, the third dimension being larger: quantum confinement is in two spatial dimensions. Such semi-conductive nanoparticles are for instance nanorods, nanowires or nanorings.

Semi-conductive nanoparticles may have a nanometric size in one dimension, the other dimensions being larger: quantum confinement is in one spatial dimension only. Such semi-conductive nanoparticles are for instance nanoplates, nanosheets, nanoribbons or nanodisks. Nanoplates are especially interesting in this disclosure because absorption cross section - *i.e.,* efficiency to capture a photon of incident light on the nanoparticle - is ten times higher than a nanosphere having the same composition and structure. This higher cross section improves significantly absorption.

The exact shape of semi-conductive nanoparticles defines confinement properties; then electronic and optical properties depending on composition of semi-conductive nanoparticle, in particular the band gap, then λₘₐₓ of the final filtering film. It has been also observed that nanoparticles with a nanometric size in one dimension, especially nanoplates, present a sharper transition between both domains of high and low absorbance as compared to nanoparticles with other shapes. Indeed, width of transition zone is enlarged if nanometric size of nanoparticles fluctuates around a mean value. When nanometric size is controlled in only one dimension, *i.e.* for nanoplates, by a strict number of atomic layers, thickness fluctuations are almost null and transition between absorbing and non-absorbing state is very sharp. This leads to particularly efficient filtering films.

In an embodiment, semi-conductive nanoparticles are homostructures. By homostructure, it is meant that the semi-conductive nanoparticle is homogenous and has the same local composition in all its volume. A homogeneous spherical semi-conductive nanoparticle (1) is illustrated in figure 1A.

In an alternative embodiment, semi-conductive nanoparticles are heterostructures. By heterostructure, it is meant that the semi-conductive nanoparticles is comprised of several sub-volumes, each sub-volume having a different composition from neighbouring sub-volumes. In a particular embodiment, all sub-volumes have a composition defined by formula (IV) disclosed above, with different parameters, *i.e.,* elemental composition and stoichiometry.

Examples of heterostructure are core/shell nanoparticles, the core (11) having any shape disclosed above. A shell (12) is a layer covering totally or partially the core. A particular example of core/shell heterostructure is a multi-layered structure comprising a core (11) and several successive shells (12, 13). For convenience, these multi-layered heterostructures are named core/shell hereafter. Core (11) and shell (12,13) may have the same shape - sphere in sphere for example - or not - sphere in plate for instance. A core/shell spherical nanoparticle is illustrated in figure 1B. A core/shell/shell spherical nanoparticle is illustrated in figure 1C. A sphere in plate nanoparticle is illustrated in figure 1D - also named a dot in plate. A core/shell nanoplate is illustrated in figure 1E.

Another example of heterostructure are core/crown nanoparticles, the core having any shape disclosed above. A crown is a band of material disposed on the periphery of the core. This heterostructure is particularly useful with cores being nanoplates and crown disposed on the edges of the nanoplate. A core/crown nanoplate is illustrated in figure 1F.

These heterostructure may have a gradient of composition from the core to the outside of the shell so that there is no precise boundary between core and shell but properties in centre of the core are different from properties on the outer boundary of shell.

In a configuration, semi-conductive nanoparticles are II-VI type and comprise a core based on cadmium, sulfur and selenium and are selected from:
- CdSe/CdS, CdSe/CdS/ZnS, CdSe/CdS/ZnSe, CdSe/CdS/ZnSe_{y}S_{(1-y)}, CdSe/ZnSe/ZnS, CdSe/ZnSeₓS₍₁₋ₓ₎/ZnS,
- CdSeₓS₍₁₋ₓ₎/ZnS, CdSeₓS₍₁₋ₓ₎/ZnSe, CdSeₓS₍₁₋ₓ₎/ZnSe_{y}S_{(1-y)}, CdSeₓTe₍₁₋ₓ₎/ZnS, CdSeₓTe₍₁₋ₓ₎/ZnSe,
- CdSe/Cd_{y}Zn_{(1-y)}S, CdSe/Cd_{y}Zn_{(1-y)}S/ZnS, CdSe/Cd_{y}Zn_{(1-y)}S/ZnSe, CdSe/Cd_{y}Zn_{(1-y)}S/ZnSe_{z}S_{(1-z)}
- CdSe/Cd_{y}Zn_{(1-y)}Se, CdSe/Cd_{y}Zn_{(1-y)}Se/ZnS, CdSe/Cd_{y}Zn_{(1-y)}Se/ZnSe, CdSe/Cd_{y}Zn_{(1-y)}Se/ZnSe_{z}S_{(1-z)},
- CdSeₓS₍₁₋ₓ₎/CdS, CdSeₓS₍₁₋ₓ₎/CdS/ZnS, CdSeₓS₍₁₋ₓ₎/CdS/ZnSe, CdSeₓS₍₁₋ₓ₎/CdS/ZnSe_{y}S_{(1-y)},
- CdSeₓS₍₁₋ₓ₎/Cd_{y}Zn_{(1-y)}S, CdSeₓS₍₁₋ₓ₎/Cd_{y}Zn_{(1-y)}S/ZnS, CdSeₓS₍₁₋ₓ₎/Cd_{y}Zn_{(1-y)}S/ZnSe, CdSeₓS₍₁₋ₓ₎/Cd_{y}Zn_{(1-y)}S/ZnSe_{z}S_{(1-z)},
- CdSeₓS₍₁₋ₓ₎/Cd_{y}Zn_{(1-y)}Se, CdSeₓS₍₁₋ₓ₎/Cd_{y}Zn_{(1-y)}Se/ZnS, CdSeₓS₍₁₋ₓ₎/Cd_{y}Zn_{(1-y)}Se/ZnSe, CdSeₓS₍₁₋ₓ₎/Cd_{y}Zn_{(1-y)}Se/ZnSe_{z}S_{(1-z)},
where x, y and z are rational numbers between 0 (excluded) and 1 (excluded).

In a configuration, semi-conductive nanoparticles are II-VI type and comprise a core based on zinc, sulfur and selenium and are selected from:
- ZnSe/ZnS, ZnSe/ZnSe_{y}S_{(1-y)}, ZnTe/ZnSe_{y}S_{(1-y)}
- ZnSeₓS₍₁₋ₓ₎/ZnS, ZnSeₓS₍₁₋ₓ₎/ZnSe, ZnSeₓS₍₁₋ₓ₎/ZnSe_{y}S_{(1-y)}, ZnSeₓTe₍₁₋ₓ₎/ZnS, ZnSeₓTe₍₁₋ₓ₎/ZnSe, ZnSeₓTe₍₁₋ₓ₎/ZnSeₓS₍₁₋ₓ₎,
- ZnSe/Cd_{y}Zn_{(1-y)}S, ZnSe/Cd_{y}Zn_{(1-y)}S/ZnS, ZnSe/Cd_{y}Zn_{(1-y)}S/ZnSe, ZnSe/Cd_{y}Zn_{(1-y)}S/ZnSe_{z}S_{(1-z)}
- ZnSe/Cd_{y}Zn_{(1-y)}Se, ZnSe/Cd_{y}Zn_{(1-y)}Se/ZnS, ZnSe/Cd_{y}Zn_{(1-y)}Se/ZnSe, ZnSe/Cd_{y}Zn_{(1-y)}Se/ZnSe_{z}S_{(1-z)},
- ZnSeₓS₍₁₋ₓ₎/ZnS, ZnSeₓS₍₁₋ₓ₎/ZnS/ZnSe, ZnSeₓS₍₁₋ₓ₎/ZnS/ZnSe_{y}S_{(1-y)},
- ZnSeₓS₍₁₋ₓ₎/Cd_{y}Zn_{(1-y)}S, ZnSeₓS₍₁₋ₓ₎/Cd_{y}Zn_{(1-y)}S/ZnS, ZnSeₓS₍₁₋ₓ₎/Cd_{y}Zn_{(1-y)}S/ZnSe, ZnSeₓS₍₁₋ₓ₎/Cd_{y}Zn_{(1-y)}S/ZnSe_{z}S_{(1-z)},
- ZnSeₓS₍₁₋ₓ₎/Cd_{y}Zn_{(1-y)}Se, ZnSeₓS₍₁₋ₓ₎/Cd_{y}Zn_{(1-y)}Se/ZnS, ZnSeₓS₍₁₋ₓ₎/Cd_{y}Zn_{(1-y)}Se/ZnSe, ZnSeₓS₍₁₋ₓ₎/Cd_{y}Zn_{(1-y)}Se/ZnSe_{z}S_{(1-z)},
where x, y and z are rational numbers between 0 (excluded) and 1 (excluded). In this configuration, x is preferably a rational number between 0 (excluded) and 0.6.

In a configuration, semi-conductive nanoparticles are II-VI type and comprise a core based on zinc, cadmium, sulfur and selenium and are selected from:
- Cd_{w}Zn_{(1-w})Se/CdS, Cd_{w}Zn_{(1-w)}Se/CdS/ZnS, Cd_{w}Zn_{(1-w)}Se/ZnSe/ZnS, Cd_{w}Zn_{(1-w)}Se/CdS/ZnSe, Cd_{w}Zn_{(1-w)}Se/CdS/ZnSe_{y}S_{(1-y)},
- Cd_{w}Zn_{(1-w)}SeₓS₍₁₋ₓ₎/ZnS, Cd_{w}Zn_{(1-w)}SeₓS(₁₋ₓ₎/ZnSe, Cd_{w}Zn_{(1-w)}SeₓS₍₁₋ₓ₎/ZnSe_{y}S_{(1-y)}, Cd_{w}Zn_{(1-w)}SeₓTe₍₁₋ₓ₎/ZnS, Cd_{w}Zn_{(1-w)}SeₓTe₍₁₋ₓ₎/ZnSe,
- Cd_{w}Zn_{(1-w})Se/Cd_{y}Zn_{(1-y})S, Cd_{w}Zn_{(1-w})Se/Cd_{y}Zn_{(1-y)}S/ZnS, Cd_{w}Zn_{(1-w)}Se/Cd_{y}Zn_{(1-y)}S/ZnSe, Cd_{w}Zn_{(1-w)}Se/Cd_{y}Zn_{(1-y)}S/ZnSe_{z}S_{(1-z)}
- Cd_{w}Zn_{(1-w)}Se/Cd_{y}Zn_{(1-y)}Se, Cd_{w}Zn_{(1-w)}Se/Cd_{y}Zn_{(1-y)}Se/ZnS, Cd_{w}Zn_{(1-w)}Se/Cd_{y}Zn_{(1-y)}Se/ZnSe, Cd_{w}Zn_{(1-w)}Se/Cd_{y}Zn_{(1-y)}Se/ZnSe_{z}S_{(1-z)},
- Cd_{w}Zn_{(1-w)}SeₓS₍₁₋ₓ₎/CdS, Cd_{w}Zn_{(1-w)}SeₓS₍₁₋ₓ₎/CdS/ZnS, Cd_{w}Zn_{(1-w)}SeₓS₍₁₋ₓ₎/CdS/ZnSe, Cd_{w}Zn_{(1-w)}SeₓS₍₁₋ₓ₎/CdS/ZnSe_{y}S_{(1-y)},
- Cd_{w}Zn_{(1-w)}SeₓS₍₁₋ₓ₎/Cd_{y}Zn_{(1-y)}S, Cd_{w}Zn_{(1-w)}SeₓS₍₁₋ₓ₎/Cd_{y}Zn_{(1-y)}S/ZnS, Cd_{w}Zn_{(1-w)}SeₓS₍₁₋ₓ₎/Cd_{y}Zn_{(1-y)}S/ZnSe, Cd_{w}Zn_{(1-w})SeₓS₍₁₋ₓ₎/Cd_{y}Zn_{(1-y)}S/ZnSe_{z}S_{(1-z)},
- Cd_{w}Zn_{(1-w)}SeₓS₍₁₋ₓ₎/Cd_{y}Zn_{(1-y)}Se, Cd_{w}Zn_{(1-w)}SeₓS₍₁₋ₓ₎/Cd_{y}Zn_{(1-y)}Se/ZnS, Cd_{w}Zn_{(1-w)}SeₓS₍₁₋ₓ₎/Cd_{y}Zn_{(1-y)}Se/ZnSe, Cd_{w}Zn_{(1-w)}SeₓS₍₁₋ₓ₎/Cd_{y}Zn_{(1-y)}Se/ZnSe_{z}S_{(1-z)},
where w, x, y and z are rational numbers between 0 (excluded) and 1 (excluded).

Most preferred II-VI nanoparticles are CdSe/CdS/ZnS, CdSeₓS₍₁₋ₓ₎/CdS/ZnS, CdSe/ZnSe/ZnS, CdSe/ZnSeₓS₍₁₋ₓ₎/ZnS, CdSeₓS₍₁₋ₓ₎/ZnSe/ZnS, CdₓZn₍₁₋ₓ₎Se/ZnSe/ZnS, ZnSeₓS₍₁₋ₓ₎/ZnS, ZnSeₓS₍₁₋ₓ₎/ZnSe, ZnSeₓS₍₁₋ₓ₎/ZnSe_{y}S_{(1-y)}, where x and y are rational numbers between 0 (excluded) and 1 (excluded).

Other particularly suitable nanoparticles are III-V type semi-conductive nanoparticles and are selected from InP/ZnS, InP/ZnSe, InP/ZnSeₓS₍₁₋ₓ), InP/CdS/ZnS, InP/ZnSe/ZnS, InP/ZnSeₓS₍₁₋ₓ₎/ZnS, InP/GaP, CuₓIn_{y}Zn_{(1-x-y)}S/ZnS, InₓAs₍₁₋ₓ₎P/ZnSeₓS₍₁₋ₓ₎, where x and y are rational numbers between 0 (excluded) and 1 (excluded).

Other particularly suitable nanoparticles are I-III-VI₂ type semi-conductive nanoparticles and are selected from AgInS₂, AgInSe₂, CuₓIn₍₁₋ₓ₎S₂, CuₓIn₍₁₋ₓ₎Se₂, CuₓAl₍₁₋ₓ)S₂, CuₓAl₍₁₋ₓ₎Se₂, especially CuInS₂, CuInSe₂, CuAlS₂ and CuAlSe₂, where x is a rational number between 0 (excluded) and 2 (excluded). These semi-conductive nanoparticles may be the core of the following core/shell heterostructures: AgInS₂/ZnSe, AgInSe₂/ZnSe, CuInS₂/ZnSe, CuInSe₂/ZnSe, CuAlS₂/ZnSe, CuAlSe₂/ZnSe, CuₓIn₍₂₋ₓ₎S₂/ZnSe, CuₓIn₍₂₋ₓ₎Se₂/ZnSe, CuₓAl₍₂₋ₓ₎S₂/ZnSe, CuₓAl₍₂₋ₓ₎Se₂/ZnSe, AgInS₂/ZnS, AgInSe₂/ZnS, CuInS₂/ZnS, CuInSe₂/ZnS, CuAlS₂/ZnS, CuAlSe₂/ZnS, CuₓIn₍₂₋ₓ₎S₂/ZnS, CuₓIn₍₂₋ₓ₎Se₂/ZnS, CuₓAl₍₂₋ₓ₎S₂/ZnS, CuₓAl₍₂₋ₓ₎Se₂/ZnS, where x is a rational number between 0 (excluded) and 2 (excluded).

Other particularly suitable nanoparticles are selected from doped quantum dots as core, such as ZnSe:Mn/ZnS, or ZnSe:Cu/ZnS.

Other particularly suitable nanoplates are selected from ZnTe/ZnSe_{y}S_{(1-y)}, ZnSeₓTe₍₁₋ₓ₎/ZnS, ZnSeₓTe₍₁₋ₓ₎/ZnSe, ZnSeₓTe₍₁₋ₓ₎/ZnSe_{y}S_{(1-y)}, where x and y are rational numbers between 0 (excluded) and 1 (excluded).

In an advantageous embodiment, semi-conductive nanoparticles have a largest dimension below 100 nm, in particular below 50 nm, ideally below 20 nm. Semi-conductive nanoparticles of small size do not induce light scattering when dispersed in a material having a different refractive index.

In an embodiment, the amount of semi-conductive nanoparticles in the filtering film is in a range from 5 wt% to 50 wt%, based on the weight of the filtering film, for a 20 µm-thick film, preferably from 10 wt% to 45 wt%, more preferably from 15 wt% to 40 wt%.

Semi-conductive nanoparticles of II-VI type and comprising a core based on zinc, sulfur and selenium are especially suitable as compounds absorbing UV-light when used with a concentration from 5 wt% to 50 wt%, preferably from 10 wt% to 45 wt%, more preferably from 15 wt% to 40 wt%, based on the weight of the filtering film, for a 20 µm-thick film.

In an embodiment, the semi-conductive nanoparticles are capped with an organic layer, an inorganic layer or a mixture thereof.

### Composite particles

In an embodiment, the semi-conductive nanoparticles are encapsulated in an encapsulating material, leading to composite particles. By encapsulating material, it is meant a material that covers all surface of semi-conductive nanoparticles. In other words, encapsulating material forms a barrier around the semi-conductive nanoparticles. Such a barrier as several advantages. In particular, said semi-conductive nanoparticles may be protected against chemicals, *e.g*., moisture, oxidants. Besides, semi-conductive nanoparticles that are not dispersible in a medium may be encapsulated in a material whose compatibility with said medium is good: the barrier behaves as a compatibilization agent. In addition, encapsulated semi-conductive nanoparticles may be under the form of a powder dispersible in a medium instead of a dispersion in a solvent, thereby providing with easier handling. Last, the encapsulating material may have a role of refractive index matching, in order to lower diffusion or haze: indeed, when semi-conductive nanoparticles are dispersed in a matrix, haze is proportional to the difference of refractive index between the matrix and the dispersed nanoparticles. Adding an encapsulating material with an intermediate refractive index mitigates this effect and lowers haze.

The encapsulating material may be an organic material or an inorganic material. For instance, the organic material may be selected from vinyl polymers, allyl polymers, (meth)acrylic polymers; epoxy compounds; polyurethane, polyester, polythiourethane materials, or mixture thereof. For instance, the inorganic material may be selected from sol gel materials, metal oxide materials, mineral oxides, or mixture thereof.

Suitable inorganic material may be selected from the group consisting of SiO₂, Al₂O₃, TiO₂, ZrO₂, HfO₂, GeO₂, SnO₂, or a mixture thereof, including for instance Al_{y}Zr_{z}O with $\frac{3}{2} y + 2 z = 1$. In an embodiment, the encapsulating material does not consist of pure SiO₂. By "pure", it is here meant that the encapsulating inorganic material is obtained by a synthesis intended to produce silica only. Impurities may be present in reactants as it is well known in the inorganic synthesis domain and still the synthesis may lead to pure silica.

In an embodiment, the encapsulating material does not absorb UV-light, and absorbance of the filtering film is only defined by semi-conductive nanoparticles. Alternatively, the encapsulating material does absorb UV-light, and absorbance of the filtering film is defined by the sum of absorbance of semi-conductive nanoparticles and absorbance of encapsulating material.

In an embodiment, the loading charge of the semi-conductive nanoparticles in the composite particle is at least 10%, preferably at least 25%, more preferably at least 50%, said loading charge being the mass ratio between the mass of semi-conductive nanoparticles comprised in a composite particle and the mass of said composite particle. Indeed, the performance of composite particles is proportional to the concentration of semi-conductive nanoparticles they contain. Therefore, a high concentration of semi-conductive nanoparticles is advantageous. It has to be noted however that increasing concentration of semi-conductive nanoparticles without degrading their properties - as a consequence of aggregation or manufacturing process for instance - is not easy.

The composite particles may be in the form of a monodisperse population. Monodisperse composite particles are advantageous for various reasons, depending on the domain of application. When composite particles are used in filtering films, a homogeneous size distribution avoids uncontrolled light diffusion and ensures spatial homogeneity of the filtering film.

**In** an embodiment, composite particles have a largest dimension below 500 nm, in particular below 300 nm, ideally below 200 nm.

The mean size of the composite particles is preferably in a range from 50 nm to 500 nm, more preferably from 50 nm to 250 nm. Composite particles having a mean size from 50 nm to 250 nm, preferably from 50 nm to 100 nm are especially suitable to obtain filtering films with high transparency and low haze.

The composite particles may be chemically modified on their surface. Chemical modification may be obtained by grafting, by adsorption of molecules or by physical processes - heat, vacuum or gaseous treatment. Chemical modification may use compatibilization agents, allowing to mix composite particles in complex formulations - such as resins, varnishes, paints, colloidal dispersion, polymerizable compositions... - without aggregation or phase separation of the composite particles.

### Organic compounds absorbing UV-light

In addition to semi-conductive nanoparticles, the filtering film optionally comprises organic anti-UV compounds.

Especially suitable organic anti-UV compounds may be selected in the group consisting of benzotriazoles, triazines, piperidines, benzophenones, catechol, their derivatives, and mixtures thereof.

Suitable benzotriazoles are derivatives of (2H-benzotriazol-2-yl)-4-hydroxybenzene such as Sodium 3-(2H-benzotriazol-2-yl)-5-sec-butyl-4-hydroxybenzenesulfonate - CAS number 92484-48-5 - or Polyethylene glycol mono-3-(3-(2H-benzotriazol-2-yl)-5-tert-butyl-4-hydroxyphenyl)-1-oxopropyl ether - CAS number 104810-48-2 - or Polyethylene glycol di[3-[3-(2H-benzotriazol-2-yl)-5-tert-butyl-4-hydroxyphenyl]-1-oxopropyl] ether - CAS number 104810-47-1 - or Benzenepropanoic acid, 3-(2H-benzotriazol-2-yl)-5-(1,1-dimethylethyl)-4-hydroxy-, C7-9-branched and linear alkyl esters - CAS number 127519-17-9 - or 2-(2H-Benzotriazol-2-yl)-6-(1-methyl-1-phenylethyl)-4-(1,1,3,3-tetramethylbutyl)phenol - CAS number 73936-91-1.

Suitable triazines are reaction products of 1,3-Benzenediol, 4-[4,6-bis(2,4-dimethylphenyl)-1,3,5-triazin-2-yl] with [(dodecyloxy)methyl]oxirane and oxirane mono[(C10-16-alkyloxy)methyl] derivatives - CAS number 153519-44-9 - or Isooctyl 2-[4-[4,6-bis[(1,1'-biphenyl)-4-yl]-1,3,5-triazin-2-yl]-3-hydroxyphenoxy]propanoate - CAS number 204848-45-3 - or triazine known as TINUVIN^{®}477 supplied by BASF.

A suitable piperidine is bis(1-octyloxy-2,2,6,6-tetramethyl-4-piperidyl) sebacate - CAS number 129757-67-1. A suitable benzophenone is 2-[4-(Diethylamino)-2-hydroxybenzoyl]benzoic Acid Hexyl Ester - CAS number 302776-68-7.

A mixture of bis(1-octyloxy-2,2,6,6-tetramethyl-4-piperidyl) sebacate - CAS number 129757-67-1 - and reaction products of 1,3-Benzenediol, 4-[4,6-bis(2,4-dimethylphenyl)-1,3,5-triazin-2-yl] with [(dodecyloxy)methyl]oxirane and oxirane mono[(C10-16-alkyloxy)methyl] derivatives - CAS number 153519-44-9 - known under tradename Eversorb AQ8 is especially suitable.

Other suitable organic anti-UV compounds are avobenzones, such as 1,3-Propanedione, 1-[4-(1,1-dimethylethyl)phenyl]-3-(4-methoxyphenyl) - CAS number 70356-09-1 - or the compound known under tradename Parsol guard.

Among these organic anti-UV compounds, those having an absorption peak - either principal or secondary - in the range from 300 nm to 340 nm are preferred. In particular, Tinuvin 384-2, Eversorb AQ8 and Tinogard HS are suitable.

Organic anti-UV compounds having an absorption peak - either principal or secondary - in the range from 340 nm to 400 nm may be used, despite their contribution to yellow appearance of the filtering film. In particular, Tinuvin^{®}Carbopotect^{®} supplied by BASF.

The following mixture of organic anti-UV compounds is also suitable: Tinuvin 384-2; Parsol Guard and Eversorb AQ8 in 1:1:1 proportion.

In an embodiment, the amount of organic anti-UV compounds in the filtering film is in a range from 2 wt% to 25 wt%, based on the weight of the filtering film, for a 20 µm-thick film, preferably from 2 wt% to 15 wt%, more preferably from 2.5 wt% to 12 wt%, even more preferably from 3 wt% to 10 wt%.

In an embodiment, the filtering film comprises a mixture of one or more semi-conductive nanoparticles and/or one or more organic anti-UV compounds. In this embodiment, the amount of compounds absorbing UV-light in the filtering film is in a range from 10 wt% to 50 wt%, based on the weight of the filtering film, for a 20 µm-thick film.

### Binder

In the disclosure, the filtering film comprises a binder. This binder may be of various polymer types, for instance selected among poly(methyl methacrylate) (PMMA), poly(butyl methacrylate), poly(lauryl methacrylate), poly(vinyl butyral), poly(vinyl acetate), poly(ethylene vinyl acetate), thermoplastic polyurethane, cellulose, ionoplast, polycarbonate, poly(ethylene vinyl alcool), polyester/melamine adducts, silicone, polyphenylmethylsiloxane, polyphenylalkylsiloxane, polydiphenylsiloxane, polydialkylsiloxane, fluorinated silicone, vinyl and hydride substituted silicone, divinylbenzene, or a mixture thereof.

The filtering film may be obtained from a thermoplastic polymer, in which compounds absorbing UV-light are dispersed, typically during melting/extrusion/stretching process. The thickness of the filtering film is here controlled by the fabrication process.

Alternatively, the filtering film may be obtained from a polymerizable composition, in which compounds absorbing UV-light are dispersed. Then, the polymerizable composition is cured - thermally, actinically (including by UV radiation) or by any other curing mean - or dried to yield a film. In this case, the polymerizable composition may comprise a solvent.

In an embodiment, the film is obtained by curing a Sol-Gel polymerizable composition and has a thickness in a range from 5 µm to 50 µm, preferably from 10 µm to 45 µm, more preferably from 15 µm to 40 µm.

In one embodiment, the film is obtained by curing a composition comprising (meth)acrylics monomers or oligomers, epoxy monomers or oligomers, or mixture thereof. In particular, the thickness of coating obtained by curing said polymerizable composition is in a range from 2 µm to 100 µm, preferably from 3 µm to 50 µm, more preferably from 4 µm to 30 µm.

### Protection provided by the filtering film

To evaluate the light filtering effect, a measure of luminous power absorbed by the photoinitiator in the radiation curable composition is relevant.

For a given illuminant, the power absorbed by the photoinitiator can be defined as the integral over the whole range of wavelength of the product of the irradiance of the illuminant *P*(*λ*) and the amount of light absorbed by the photoinitiator, as defined in equation (II): ${\int }_{300}^{500} P \left(λ\right) ⋅ \left(1-{10}^{- {A}_{φ} \left(λ\right)}\right) dλ$ (II)

The illuminant may be selected from various known sources, such as sun - represented by solar irradiance -, or artificial sources like D65 standard, illuminant, LEDs, thermal lamps, spectral lamps, ...

When the filtering film is interposed between the illuminant and the curable composition, the spectrum of light transmitted through the filtering film is defined by the product of the irradiance of the illuminant and the absorption of the filtering film: *P*(*λ*) *· 10^{-A_{ff}(λ)}.* Everything happens as if the illuminant were changed in another one: a filtered illuminant.

Finally, the power absorbed by the photoinitiator when exposed to the filtered illuminant is defined by equation (III) ${\int }_{300}^{500} P \left(λ\right) ⋅ {10}^{- {A}_{ff} \left(λ\right)} ⋅ \left(1-{10}^{- {A}_{φ} \left(λ\right)}\right) dλ$ (III) And the ratio α of equation (III) divided by equation (II) yields the lowering of exposure of the photinitiator when the filtering film is used. $α = \frac{{\int }_{300}^{500} P \left(λ\right) ⋅ {10}^{- {A}_{ff} \left(λ\right)} ⋅ \left(1-{10}^{- {A}_{φ} \left(λ\right)}\right) dλ}{{\int }_{300}^{500} P \left(λ\right) ⋅ \left(1-{10}^{- {A}_{φ} \left(λ\right)}\right) dλ}$ A ratio α less than 0.05 means that more than 95% of power over the active range of wavelengths of the illuminant is filtered out by the film. A ratio α less than 0.01 means that more than 99% of power of the active range of wavelengths of the illuminant is filtered out by the film. A ratio α less than 0.005 means that more than 99.5% of power over the active range of wavelengths of the illuminant is filtered out by the film.

According to the disclosure, the ratio α is less than 0.05. Preferably, the ratio α is less than 0.02, more preferably less than 0.01, ideally less than 0.005.

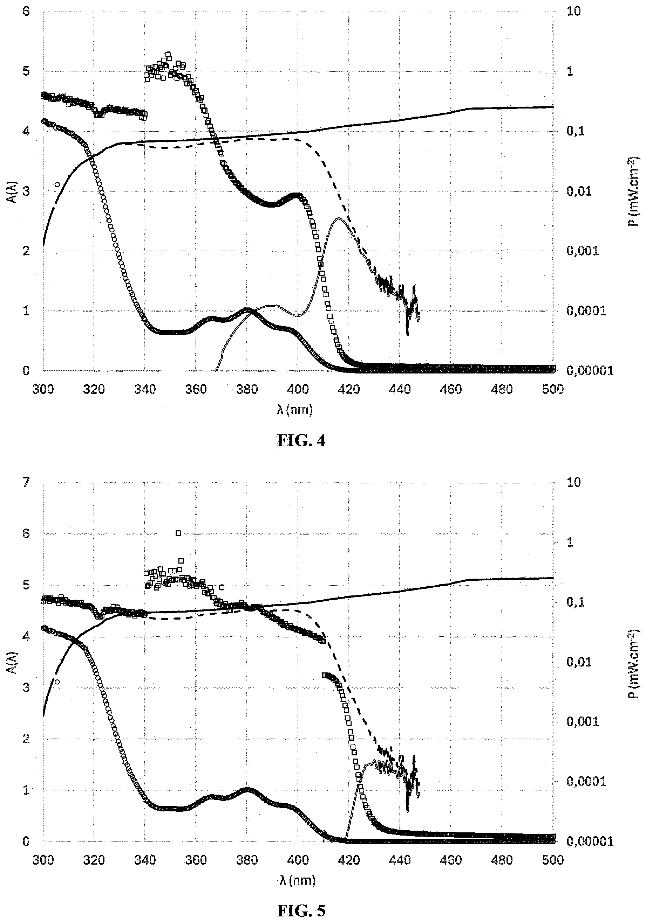

Figure 4 illustrates this principle and shows:
- The solar irradiance (at sea level, noted AM1.5). Over the range of 300 nm to 450 nm, irradiance is roughly 13 mW.cm⁻²; (solid line, right axis - logarithmic scale)
- The absorption curve of Top-Gel of example 1 (circles, left axis)
- The absorption curve of the filtering film of example 1 (squares, left axis).
One can read that the power absorbed by the photoinitiator without filtering film (dotted line, right axis) directly exposed to sun - is about 5.6 mW·cm⁻². When the filtering film is placed between the sun and the curable composition, the power absorbed by the photoinitiator (double line, right axis, almost zero over the whole range) becomes 47 µW·cm⁻². Absorption is reduced by a factor of 130, corresponding to α=0.0076.

Figure 5 illustrates the same photoinitiator but protected by the filtering film of example 2. When the filtering film is placed between the sun and the curable composition, the power absorbed by the photoinitiator becomes 4.2 µW·cm⁻². Absorption is reduced by a factor of 1500, corresponding to α=0.00067. Note that the absorption curve of the filtering film shows discontinuities due to range changes in measuring devices when absorbance becomes very high. This artefact has a negligible impact on the computation detailed here.

The filtering film may be laid on a predetermined surface of the glass container, *i.e.,* not on the whole surface. Outside this predetermined surface, the glass container has an opaque treatment, preferably an opaque coating. By opaque, it is here understood that no light, neither visible nor in the UV/HEV range, can go through the coating. In this arrangement, a predetermined surface of the glass container allows direct visualization of the colour of the radiation curable composition by a user. The predetermined surface is like a window. Outside this window, the opaque treatment ensures protection against UV/HEV light.

### Colour of the cosmetic bottle

In this disclosure, the cosmetic bottle comprises a container on which a filtering film is coated. Preferably, the container is a glass container. It is preferred that the aesthetic appearance of the container is not altered by the filtering film, in particular in terms of perceived colour and lightness.

Indeed, the container may be a "white" container, *i.e.* a container having almost no colour and transparent. In terms of colorimetry, such a container has high lightness and a low chroma. Alternatively, the container may be coloured but still transparent, with a high lightness and a faint or vivid colour - as described by chroma and hue.

In addition, the filtering film should not be hazy. Indeed, the clear appearance of the container must also be unaltered.

In an embodiment, the filtering film is transparent, preferably transparent and uncoloured.

The colour of the container is determined by well-known colorimetric measurements. A part of the container is illuminated with a standard illuminant and light transmitted through the container is analyzed according to CIE L*a*b* model (standard observer, 2°), yielding Lightness L*u and the value of b*u - u stand for uncoated container - as an estimate for the yellow appearance of the bottle. For white glass containers, b*u is almost zero. But for complex containers, with thick walls, angular shapes and/or high curvatures, multiple light reflections and optical effects may lead to slight yellow appearance, with b*u greater than 3, even greater than 5, sometime greater than 10. For colour appreciation, focusing on b* parameter is appropriate in this case, as filtering film has a limited impact on visible wavelength leading to absorption in blue range, thus yielding a residual yellow color.

In addition, measure of luminous transmittance - noted Tv hereafter - through the container gives an indication of Lightness. Indeed, a container with a low chroma will appear grey if Tv is low and bright/clear if Tv is high.

After coating with the filtering film, the container becomes a cosmetic bottle, whose colorimetric properties can be assessed again: Lightness L*c and value b*c are measured - c stands for cosmetic bottle.

In an embodiment, the difference of lightness between the cosmetic bottle and the container is lower than 5. In particular, the difference of lightness may be lower than 4, preferably lower than 3, more preferably lower than 2.

In an embodiment, the difference Δb*=|b*c-b*u| of the parameter b* of the glass container - b*u - and the glass container with the filtering film - b*c - is lower than 20, preferably lower than 15, more preferably lower than 10.

In an embodiment, the parameter b*c of the cosmetic bottle is lower than 20, preferably lower than 15. Even if value may appear large, colour balancing additives may be added to the filtering film to lower chroma but with a lowering of lightness.

In a particular embodiment, the difference of lightness between the cosmetic bottle and the container is lower than 2 and the parameter b*c of the cosmetic bottle is lower than 20.

In an embodiment, the luminous transmittance of the cosmetic bottle is greater than 90%, preferably greater than 95%, of the luminous transmittance of the container. In these conditions, the lightness of the container is not degraded by the filtering film.

If the cosmetic bottle is not evenly coloured, being decorated with a pattern or an image for instance, then the colour assessment is made on a part of the cosmetic bottle where colorimetric parameters are well defined.

### Optimum compromise

The trade off between cure inhibition and unwanted colouration of the cosmetic bottle is determined by the exact shape of the absorption curve A_{ff}(λ). Indeed, maximum absorption governed by the peak of excitonic absorption should be high enough to provide a good filtering effect of UV/HEV light, without filtering out significant visible light in the blue range. Therefore, the exact shape of the absorption curve A_{ff}(k) after the maximum is critical: a very sharp transition from absorption to non-absorption is much desired. To this end, using a single type of semi-conductive nanoparticles is preferred. Indeed, when several types of semi-conductive nanoparticles are used in mixtures, the absorption curve A_{ff}(k) is the sum of the contributions of each type of semi-conductive nanoparticles, each having a specific wavelength of transition, and finally leading to a wider maximum absorbance peak and a wider decreasing zone. Such widening degrades the desired compromise.

The optimum results are obtained with a single type of semi-conductive nanoparticles having a wavelength of transition λ_{cut} comprised in a range from 405 nm to 410 nm, in a filtering layer providing an absorbance greater than 3 at the maximum peak of absorption - illustrated in examples 2, 4, 15 and 16. This optimum formulation has proven high efficiency for diphenyl(2,4,6-trimethylbenzoyl)phosphine oxide and for 2,4,6-trimethylbenzoylethoxyphenylphosphine oxide as photoinitiators.

### BRIEF DESCRIPTION OF THE DRAWINGS

**Figure 1A-1F** illustrates various nanoparticles with homostructure (A) or heterostructures: spherical core/shell (B), spherical core/shell/shell (C), dot in plate (D), nanoplate core/shell (E) and nanoplate core/crown (F).
**Figure 2** shows the generic absorbance of a filtering film comprising semi conductive nanoparticles (logarithm scale) as a function of wavelength of light from 350 nm to 780 nm (linear scale): A_{φ}(λ) and the principle of determination of λ_{cut}.
**Figure 3** is a graph showing absorption curves A_{φ}(λ) for two photoinitiators, TPO - solid line - and TPO-L - dotted line as a function of wavelength λ (nm).
**Figure 4** is a graph showing the absorbance (left axis) of a photoinitiator (circles) and the filtering film (squares) of example 1, with the irradiance of the sun (solid line), the power absorbed by the photoiniator (dotted line) and the power absorbed by the photoinitiator when the filtering film is applied on the glass container (double line). Note the logarithmic scale for powers (right axis).
**Figure 5** is a graph similar to figure 4, in which the filtering film (squares) corresponds to example 2.

### EXAMPLES

The present disclosure is further illustrated by the following examples.

A commercial nail polish has been used, referenced Top-Gel hereafter. The absorption curve of Top-Gel is shown in figure 3 - solid line. The curve clearly shows that Top-Gel is uncoloured: the absorption curve of Top-Gel is essentially the absorption curve A_{φ}(λ) of the photoinitiaor. Besides, the photoinitiator has a cleaving maximum λ_{clm} around 380 nm - the shape of the absorption curve is a signature of diphenyl(2,4,6-trimethylbenzoyl)phosphine oxide, referenced TPO hereafter. The concentration of TPO is evaluated at 1000 ppm. Top-Gel is stored in a glass bottle without any filtering film and referenced Bottle 0. A film of Top-Gel laid on a substrate and exposed to the sun cures in about 5 s.

Bottles 1a and 1b are similar glass bottles on which a filtering film is laid. The filtering film is 20 µm thick and comprises 29wt% of a ZnSe/ZnS nanoparticle - diameter of core 3.6 nm, thickness of shell 0.6 nm - and 71wt% of a binder - polyester resin (75 parts) and hexamethoxymethyl melamine (25 parts). The absorption curve A_{ff}(k) of the filtering film is shown in figure 4 - squares. The wavelength of transition λ_{cut} is about 405 nm, and the peak of excitonic absorption is at λₘₐₓ =400 nm - with Aₘₐₓ =3. Bottle 1a is filled with the Top-Gel composition. Bottle 1b is used as an enclosure in which a glass lamella coated with a 20 µm-thick layer of Top-Gel composition is stored.

Bottles 2a and 2b are similar glass bottles on which a filtering film is laid. The filtering film is 20 µm thick and comprises 30wt% of a ZnSe/ZnS nanoparticle - diameter of core 4.1 nm, thickness of shell 0.8 nm - and 70wt% of a binder - polyester resin (75 parts) and hexamethoxymethyl melamine (25 parts). The absorption curve A_{ff}(k) of the filtering film is shown in figure 5 - squares. The wavelength of transition λ_{cut} is about 415 nm, and the peak of excitonic absorption is at λₘₐₓ =410 nm - with Aₘₐₓ =3.3. Bottle 2a is filled with the Top-Gel composition. Bottle 2b is used as an enclosure in which a glass lamella coated with a 20 µm-thick layer of Top-Gel composition is stored.

The three types of bottles are visually compared: Bottle 0 and Bottles 1 (b*=3) have no difference in perceived colour. Bottles 2 presents a very slight yellowish appearance (b*=9) but considered not relevant for the perception of the colour of Top-Gel by a consumer.

The three types of bottles are exposed to sun, whose irradiance is shown on figures 4 and 5 - solid line, according to ISO 9845-1:2002 standard.

Figure 4 shows the power absorbed by the photinitiator in Bottle 0 - according to equation (II), dotted line, as a reference - and in Bottles 1 - according to equation (III), double line - as a function of wavelength (λ). A very small amount of light is absorbed by the photoinitiator in Bottle 1, mainly around 415 nm, which is the lowest energy that can be captured by the photoinitiator, less likely to induce cleavage in free radicals.

Figure 5 shows the power absorbed by the photinitiator in Bottle 0 - according to equation (II), dotted line, as a reference - and in Bottles 2 - according to equation (III), double line - as a function of wavelength (λ). Here, almost no light is absorbed by the photoinitiator in Bottles 2.

The total power absorbed by the photoinitiator corresponds to the area below the curve and reaches 5.6 mW·cm⁻² in Bottle 0.

With filtering films, the total power absorbed by the photoinitiator is reduced to 47 µW·cm⁻² for Bottles 1 - corresponding to α=0.0076 - and 4.2 µW·cm⁻² for Bottles 2 - corresponding to α=0.00067.

The three bottles have been exposed to a SUNTEST cabin under constant illumination corresponding to D65 illuminant - thus including UV light from 300 nm wavelength - with 550W/m² total power at 40°C temperature. In Bottle 0, formation of a dry layer on top of the curable composition was observed after 60 min, together with a cloudy appearance. In Bottle 1a and Bottle 2a cloudy appearance was not observed after 3600 min.

The same experiment is reproduced with exposition to sunlight through a glass window. In Bottle 0, formation of a dry layer on top of the curable composition was observed after 6 days, together with a cloudy appearance. In Bottle 1a and Bottle 2a cloudy appearance was not observed after 100 days.

In Bottle 1b and Bottle 2b, the thin layers of curable composition were cured after 2 to 3 minutes exposure time, while an unprotected thin layer was cured in 5 s. The time ratio measured here appears to be about 30, much less than the estimated time ratio of 100 or more with filled bottles. This difference may be understood due to a difference in power density and curable composition formulation. Indeed, the curable composition usually contains free radical scavengers - or inhibitors - intended to inactivate free radical, for two reasons: first to avoid cure during storage, second to give some time to the user to lay the radiation curable composition before it cures upon exposure to natural light. The balance between free radical creation by excitation of photoinitiators and inhibition is governed by overall power applied to the curable composition. In low power conditions - typically the lighting conditions in a shop where products are exposed for sale -, inhibitors do capture free radicals as soon as they are produced: cure does not start until all inhibitors are reacted. In high power conditions, the rate of production of free radicals is so high that inhibitors cannot prevent polymerization: cure does start when inhibitors are still present in the curable composition! Therefore, time ratios observed in high power conditions - *i.e.,* SUNTEST - are less than time ratios observed in low power conditions.

In other words, for a low power of illumination, the inverse of α is a protection factor, defining the extended protection of the curable composition. With a protection factor of 20 - α=0.05 - the curable composition may be stored in a container with a filtering film 20 times longer than an unprotected curable composition, before curing occurs inside the bottle. The protection factor is intrinsic to the filtering film and well adapted to characterize pot life of radiation curable compositions.

In the following, the storage improvement is always defined as the ratio between the curing time of a layer of radiation curable composition exposed to natural sunlight and the curing time of the same layer of radiation curable composition protected by a filtering film in low power conditions. The storage improvement is a function of protection factor and power of illumination.

The same experiment is reproduced with a commercial red nail varnish - referenced RED hereafter - in which the photoinitiator is 2,4,6-trimethylbenzoylethoxyphenylphosphine oxide, referenced TPO-L hereafter. The absorption curve of TPO-L is slightly shifted towards high wavelengths as compared to TPO. Absorption curves for TPO-L - 0.1wt% in ethanol, dotted line - is shown in figure 3.

The three bottles are visually compared: Bottle 0 and Bottles 1 (b*=3) have no difference in perceived colour. Bottles 2 presents a very slight colour difference (b*=9) with Bottle 0 but considered not relevant for the perception of the colour of RED by a consumer.

The total power absorbed by the photoinitiator of RED reaches 4.0 mW·cm⁻² in Bottle 0. With filtering films, the total power absorbed by the photoinitiator is reduced to 270 µW·cm⁻² for Bottles 1 - corresponding to α=0.068 - and 4 µW·cm⁻² for Bottles 2 - corresponding to α=0.0075. With RED, Bottle 1a is not protective enough, whereas Bottle 2a provides very good protection. Indeed, the residual absorbance of TPO-L above 420 nm is not prevented by Bottle 1a, and curing is not totally prevented. On the contrary, Bottle 2a prevents efficiently photoinitiation of RED.

Further experiments are conducted with different filtering films, according to table I below.

**Table I**

| Ex | Semi-conductive Nanoparticles - organic UV absorber | wt% | Curable composition | Thickness (µm) | Storage improvement | Δb* |
|---|---|---|---|---|---|---|
| 1 | SC#1 | 29 | Top-Gel | 20 | >100 | 3 |
| 2 | SC#2 | 30 | Top-Gel | 20 | >1000 | 9 |
| 3 | SC#1 | 29 | RED | 20 | 15 | 3 |
| 4 | SC#2 | 30 | RED | 20 | 1000 | 9 |
| 5 | SC#1 | 14.5 | Top-Gel | 20 | 330 | 10 |
| | SC#2 | 15 | | | | |
| 6 | SC#1 | 14.5 | Top-Gel | 20 | 350 | 11 |
| | SC#2 | 15 | | | | |
| | T384-2 | 4.0 | | | | |
| 7 | SC#1 | 29 | Top-Gel | 20 | 470 | 12 |
| | SC#2 | 15 | | | | |
| | T384-2 | 4.0 | | | | |
| 8 | SC#1 | 29 | Top-Gel | 20 | 500 | 13 |
| | SC#2 | 15 | | | | |
| | T384-2 | 16.0 | | | | |
| 9 | SC#1 | 29 | Top-Gel | 20 | 460 | 12 |
| | SC#2 | 15 | | | | |
| | Uv A+ | 4.0 | | | | |
| 10 | SC#1 | 29 | Top-Gel | 20 | 510 | 13 |
| | SC#2 | 15 | | | | |
| | Uv A+ | 16.0 | | | | |
| 11 | SC#1 | 14.5 | Top-Gel | 20 | 340 | 10 |
| | SC#3 | 15 | | | | |
| 12 | SC#1 | 14.5 | Top-Gel | 20 | 360 | 11 |
| | SC#4 | 15 | | | | |
| 13 | SC#1 | 14.5 | Top-Gel | 20 | 370 | 12 |
| | SC#5 | 15 | | | | |
| 14 | SC#1 | 14.5 | Top-Gel | 20 | 300 | 8 |
| | SC#6 | 15 | | | | |
| 15 | SC#3 | 29 | Top-Gel | 20 | >100 | 6 |
| 16 | SC#6 | 30 | Top-Gel | 20 | >100 | 5 |

The semi-conductive nanoparticles used are the following:
- SC#1: ZnSe/ZnS nanoparticle - diameter of core 3.6 nm, thickness of shell 0.6 nm - λ_{cut}=400 nm.
- SC#2: ZnSe/ZnS nanoparticle - diameter of core 4.1 nm, thickness of shell 0.8 nm - λ_{cut}=410 nm.
- SC#3: ZnSeS/ZnS nanoparticle - diameter of core 3.7 nm, thickness of shell 0.75 nm - λ_{cut}=405 nm.
- SC#4: ZnSe/ZnS nanoparticle - diameter of core 4.7 nm, thickness of shell 0.8 nm - λ_{cut}=415 nm.
- SC#5: ZnSe/ZnS nanoparticle - diameter of core 5.4 nm, thickness of shell 0.8 nm - λ_{cut}=420 nm.
- SC#6: ZnSeₓS₍₁₋ₓ₎ nanoparticle with x about 0.94 - diameter of core 3.0 nm, thickness of shell 1.3 nm.

The organic UV absorber used in examples 1-16 are referenced as follow:

| Ref | Tradename | CAS | Absorbing molecules |
|---|---|---|---|
| T384-2 | Tinuvin 384-2 | 127519-17-9 | Benzenepropanoic acid, 3-(2H-benzotriazol-2-yl)-5-(1,1- dimethylethyl)-4-hydroxy-, C7-9-branched and linear alkyl esters |
| Uv A+ | Uvinul A plus | 302776-68-7 | 2-[4-(Diethylamino)-2-hydroxybenzoyl]benzoic Acid Hexyl Ester |

## Claims

1. A cosmetic bottle comprising
• a glass container,
• a radiation curable composition in the glass container, said radiation curable composition comprising at least 500 ppm of a photoinitiator having a cleaving maximum λ_{clm} in a range from 350 nm to 400 nm, and an absorption function A_{φ}(λ); and
• a filtering film on the glass container, said filtering film comprising semi-conductive nanoparticles absorbing UV-light, said filtering film having an absorption function A_{ff}(k) and a wavelength of transition λ_{cut} in a range from 370 nm to 420 nm;
wherein the filtering film lowers by 95% or more the power absorbed by the photoinitiator when exposed to a light source having an irradiance P(λ), as defined by ratio α in equation (I) $α = \frac{{\int }_{300}^{500} P \left(λ\right) ⋅ {10}^{- {A}_{ff} \left(λ\right)} ⋅ \left(1-{10}^{- {A}_{φ} \left(λ\right)}\right) dλ}{{\int }_{300}^{500} P \left(λ\right) ⋅ \left(1-{10}^{- {A}_{φ} \left(λ\right)}\right) dλ} \leq 0.05$ (I)

2. The cosmetic bottle according to claim **1,** wherein the photoinitiator is selected from the group consisting of phosphinate or phosphine oxide.

3. The cosmetic bottle according to claim **2,** wherein the photoinitiator is selected from the group consisting of 2,4,6-trimethylbenzoylethoxyphenylphosphine oxide, diphenyl(2,4,6-trimethylbenzoyl)phosphine oxide, phenylbis(2,4,6-trimethylbenzoyl)phosphine oxide, bis(2,6-dimethoxybenzoyl)(2,4,4-trimethylpentyl)phosphine oxide, and mixtures thereof.

4. The cosmetic bottle according to any one of claim **1** to **3,** wherein the semi-conductive nanoparticles have a formula
MₓE_{y} (IV),
wherein:
M is selected from the group consisting of Zn, Cd, Hg, Cu, Ag, Al, Ga, In, Si, Ge, Sn, Pb or a mixture thereof;
E is selected from the group consisting of S, Se, Te, N, P, As, Sb, or a mixture thereof;
x and y are independently a decimal number from 0 to 5; and
x and y are not simultaneously equal to 0.

5. The cosmetic bottle according to claim **4,** wherein the semi-conductive nanoparticles comprise a material selected from the group consisting of CdS, CdSe, CdTe, ZnS, ZnSe, ZnTe, HgS, HgSe, HgTe, GeS, GeSe, GeTe, SnS, SnSe, SnTe, PbS, PbSe, PbTe, GeS₂, GeSe₂, SnS₂, SnSe₂, CuInS₂, CuInSe₂, CuAlS₂, CuAlSe₂, CuInZnS, CuInZnSe, AgInS₂, AgInSe₂, CuS, Cu₂S, Ag₂S, Ag₂Se, Ag₂Te, FeS, FeS₂, InP, Cd₃P₂, Zn₃P₂, AIN, AlP, AlAs, AlSb, GaN, GaP, GaAs, GaSb, InN, InP, InAs, InSb, InAsP, or a mixture thereof; preferably selected from the group consisting of CdS, CdSe, CdTe, ZnS, ZnSe, ZnTe, or a mixture thereof.

6. The cosmetic bottle according to claim **4,** wherein the semi-conductive nanoparticles are core/shell nanoparticles selected from the group consisting of ZnSeₓS₍₁₋ₓ₎/ZnSe_{y}S_{(1-y)}, where x and y are rational numbers between 0 (included) and 1 (included).

7. The cosmetic bottle according to any one of claim **1** to **6,** wherein the ratio α is less than 0.02, preferably less than 0.01, more preferably less than 0.005.

8. The cosmetic bottle according to any one of claim **1** to **7,** wherein the absorbance A_{ff}(k) of the filtering film has:
• a local maximum absorbance of highest wavelength in the range from 370 nm to 420 nm, said local maximum having an absorbance value Aₘₐₓ for a wavelength λₘₐₓ,
• a value of 0.9Aₘₐₓ for a wavelength λ_{0.9}, λ_{0.9} being greater than λₘₐₓ;
• a value of 0.5Aₘₐₓ for a wavelength λ₀.₅, λ_{0.5} being greater than λ_{0.9}; and
• |λ_{0.5} - λ_{0.9}| is less than 15 nm.

9. The cosmetic bottle according to any one of claim **1** to **8,** wherein the weight concentration of the semi-conductive nanoparticles in the filtering film is in a range from 5 wt% to 50 wt%, based on the weight of the filtering film, for a 20 µm-thick film.

10. The cosmetic bottle according to any one of claim **1** to **9,** wherein the semi-conductive nanoparticles are encapsulated in an encapsulating material, thereby forming composite particles.

11. The cosmetic bottle according to any one of claim **1** to **10** wherein the filtering film is transparent, preferably transparent and uncoloured.

12. The cosmetic bottle according to any one of claim **1** to **11,** wherein the glass container has a colour (L*u, b*u) and the difference of lightness between the glass container and the glass container with the filtering film is lower than 5.

13. The cosmetic bottle according to claim **12,** wherein the difference Δb*=|b*c-b*u| of the parameter b* of the glass container - b*u - and the glass container with the filtering film - b*c - is lower than 20, preferably lower than 15.

14. The cosmetic bottle according to any one of claim **1** to **13,** wherein the radiation curable composition is a nail varnish, a nail polish or a gel nail coating.

15. The cosmetic bottle according to any one of claim **1** to **14,** wherein the glass container carries the filtering film over a predetermined surface and carries an opaque treatment outside said predetermined surface.
